# EUROPEAN PATENT SPECIFICATION

(11) **EP 0 521 373 B1**
(45) Date of publication and mention of the grant of the patent: **07.02.1996**
(21) Application number: 92110558.1
(22) Date of filing: 22.06.1988
(51) Int. Cl.: C07D 307/92, C07D 303/16, C07C 69/63

(54) **Method for preparing dodecahydro-3a,6,6,9a-tetramethylnaphtho[2,1-b]furan**
Verfahren zur Herstellung von Dodecahydro-3a,6,6,9a-tetramethylnaphto(2,1-b)furan
Procédé pour la préparation de docécahydro-3a,6,6,9a-tétraméthylnaphto(2,1-b)furanne

(30) Priority: 23.06.1987 US 65426
(43) Date of publication of application: 07.01.1993
(62) Divisional of application: 88109936.0
(73) Proprietor: GIVAUDAN-ROURE (INTERNATIONAL) S.A., CH-1214 Vernier, Genève (CH)
(72) Inventor: Christenson, Philip A., Midland Park, New Jersey 07432 (US)
(74) Representative: VOSSIUS & PARTNER

(56) References cited:
- EP-A- 0 170 955
- HELVETICA CHEMICA ACTA vol. 33, no. 161, 1 August 1950, pages 1251 - 1260; M.STOLL ET AL.: 'Odeur et Constitution III.Les substances bicyclohomofanésiques'
- JOURNAL OF CHEMICAL RESEARCH (S) 1981, pages 236 - 237; N.BENSADOUN ET AL.: 'Stereoelectronic Control in the beta-Fragmentation Reactions of Alkoxy Radicals.An Empirical Predictive Rule' Transformation 9-10
- TETRAHEDRON LETTERS vol. 26, no. 46, 1985, GREAT BRITAIN pages 5717 - 5720; J.DE PASCUAL TERESA ET AL.: 'Functionalization at C-12 in Labdanic Diterpenes:Synthesis of the Natural Diterpenic Lactones isolated from Cistus Ladaniferus L.' Transformation Ib-IV
- JOURNAL OF THE CHEMICAL SOCIETY,CHEMICAL COMMUNICATIONS no. 14, 20 July 1977, LONDON pages 478 - 479; ZDENKO HAMERSAK ET AL.: 'Hypoiodite Thermolysis-cyclization Reaction.Convenient Synthesis of 4-Homoprotoadamantan-4-0ne (Tricyclo[5.3.1.0]undecan-4-one) from 3-Homoadamantanol.' Transformation 1-2-4
- 'Houben Weyl: Methoden der Organischen Chemie vol.IV/b' 1975 , GEORG THIEME VERLAG , STUTTGART
- SYNTHESIS September 1984, STUTTGART pages 709 - 726; A.VARVOGLIS: 'Polyvalent Iodine Compounds in Organic Synthesis'

## Description

The compound dodecahydro-3a,6,6,9a-tetramethylnaphtho[2,1-b]furan (hereinhafter Naphthofuran II) is a synthetic congener of Ambergris, a rare perfumery composition of natural origin having a sweet, woody, amber bouquet. Naphthofuran II has been used in perfume compositions and in cleaning formulations, such as fragrances for toiletries and household products, where a persistent amber effect is desired. Naphthofuran II is also a component of tincture of Ambergris and synthetic Naphthofuran II has been used in artificial Ambergris formulations.

Naphthofuran II may be produced synthetically from 3-ethylenyldecahydro-2-hydroxy-α-2,5,5,8a-pentamethyl-1-naphthalenepropanol, commonly known as sclareol. One method for this conversion is a two stage oxidation followed by a hydride reduction and cyclization. See, for example, U.S. Patent No. 3,050,532; U.S. Patent No. 3,029,255; and Aust. J. Chem., 1971, 24, 591. Another method involves oxidation of sclareol to sclareol oxide followed by a multi-step synthesis to produce Naphthofuran II as a mixture of stereoisomers. See, for example, Helv. Chim. Acta, 1942, 25, 621; J. Chem. Soc., 1960, 4613; Helv. Chim. Acta, 1950, 33, 1251; U.S. Patent 3,029,255, 1962; Helv. Chim. Acta, 1950, 33 1308; J. Am. Chem. Soc., 1963, 85, 3979); and Helv. Chim. Acta, 1951, 34, 1664.

These methods, however, lack simplicity and are based upon multiple synthetic reactions. Moreover, they produce undesirable side reactions and do not generate high yields of Naphthofuran II. Accordingly, it is an object of the invention to develop a simple method for the production of Naphthofuran II from sclareol. Another object is the production of a high yield of Naphthofuran II without the complications caused by side reactions.

These and other objects are achieved by the invention which is directed to a method for the production of Naphthofuran (II) from sclareol. In this method, an alkoxy-radical fragmentation reaction is used to convert sclareol derivatives to a 9-haloethyl decalin derivative of the formula I 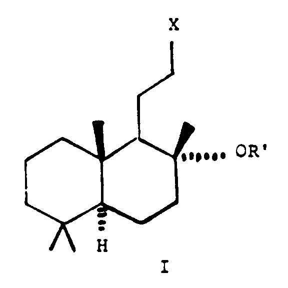
wherein X is iodo and R' is an alkanoyl group of 2 to 5 carbons. Basic hydrolysis of this 9-iodoethyl decalin derivative produces Naphthofuran of the formula II 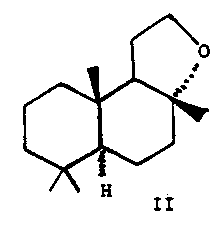
According of the method of the invention, a sclareol alkanoate derivative of the formula III 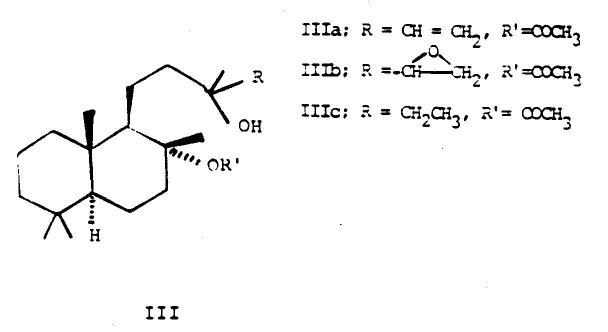
wherein R is vinyl, epoxyethyl or ethyl, R' is an alkanoyl group of 2 to 5 carbons and R' is alkanoyloxy of 2 to 5 carbons is treated with iodine and an oxidizing agent to produce the decalin derivative of formula I wherein X is iodo.

The invention is directed as well to a method for preparing the novel 9-haloethyl decalin derivatives of formula I and an epoxy-sclareol acetate (IIIb).

The process of the present invention produces a high yield of dodecahydro-3a,6,6,9a-tetramethylnaphtho[2,1-b]furan (Naphthofuran II) from sclareol derivatives through a sequence of steps which employ an alkoxy-radical fragmentation reaction and a hydrolysis. It avoids production of side products that could alter the fragrance qualities of Napthofuran II.

The alkoxy-radical reaction employed in the process of the invention, the sclareol alkanoate derivative of formula III (see foregoing Summary) can be converted to the decalin derivative of formula I (see foregoing Summary) wherein X is iodide by treatment with iodine and an oxidant in an inert solvent. The oxidant can be mercuric oxide, lead tetraacetate, iodosobenzene diacetate, periodinane iodosobenzene or iodoxybenzene. Preferred oxidants include iodosobenzene, lead tetraacetate and iodosobenzene diacetate, the latter two being especially preferred. The amount of oxidant used may be from about 50 to about 250 mole percent, about 50 to about 200 mole percent being preferred and about 100 to about 200 mole percent being especially preferred, the mole percentage being measured relative to the molar amount of the sclareol alkanoate derivative present. The reaction may be performed in an inert solvent, such as an aromatic hydrocarbon, an aliphatic hydrocarbon or a halocarbon. Preferred solvents are benzene, toluene, chlorobenzene, xylene, hexane, cyclohexane, dichloroethylene, tetrachloroethane, and carbon tetrachloride. The especially preferred solvents include benzene, carbon tetrachloride and chlorobenzene, with benzene being particularly preferred. The reaction is usually performed in the presence of an acid scavenger such as an amine or an alkali or alkaline earth metal carbonate. Calcium carbonate, pyridine, sodium carbonate and triethyl amine are preferred acid scavengers. The reaction may be conducted at a temperature range of about 60°C to about 130°C with about 70°C to about 120°C being preferred and about 75°C to about 85°C being most preferred.

The iodoethyl decal in derivatives of formula 1 produced in the foregoing fashion are converted to Naphthofuran II in high yield according to the process of the present invention by hydrolysis with a metal hydroxide in a solvent therefor. Alkali or alkaline earth metal hydroxides are preferred. Potassium hydroxide is the most preferred. A variety of protic or aprotic solvents mixed with water may be used, such as lower alkyl alcohols, ethyl ether, tetrahydrofuran, dioxane, dimethoxyethane, dimethylsulfoxide, ethylene glycol dimethylformamide, N-methylpyrrolidone or acetonitrile. The preferred solvents are a mixture of water and alcohols or diols, such as, methanol, ethanol, propanol, isopropanol, n-butanol, t-butanol, ethylene glycol. The most preferred solvent is a mixture of water and isopropanol. The reaction may be carried out in the temperature range of about 25° to about 150°C. The preferred temperature range is about 50° to about 100°C. The most preferred temperature range is about 70° to about 85°C.

The process of the present invention may be applied to other labdane derivatives such as manool(VI), larixol (VII), torulosol (VIII) and cupressic acid (IX) to provide compounds of general formula X, wherein R and R' are as described in VI-VIII and X is halogen. 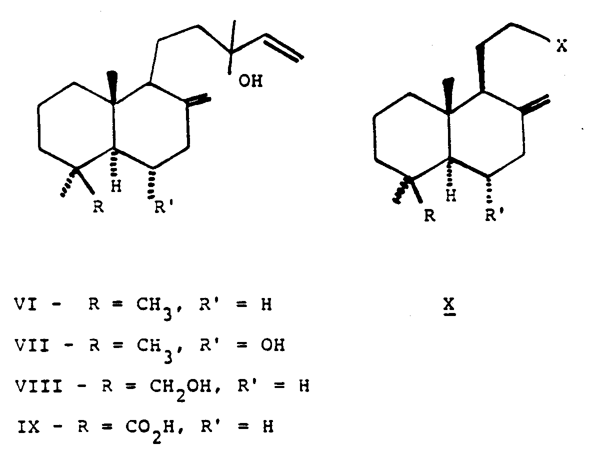
Compounds X may be converted into useful terpenoid derivatives.

Practice of other embodiments of the invention will be apparent from the foregoing discussion. The alkoxy-radical fragmentation can be applied pursuant to reaction procedures disclosed by Waegell et al., J. Chem. Res. S., 1981, 236. The following examples illustrate some further embodiments of the invention.

### Experimental Section

General. Benzene was dried over 4A molecular sieves prior to use. Sclareol, purchased from R.J. Reynolds Tobacco Co., was recrystallized from hexane prior to use. All other reagents and solvents were of reagent grade and were used as received.

IR spectra were obtained with a Perkin-Elmer 710B spectrophotometer. Routine ¹H-NMR spectra were recorded with a Varian Associates T-60A NMR instrument. The ¹H-NMR spectral data reported at 250 MHz were recorded on a Bruker WM250 NMR instrument and the ¹³C-NMR spectra were obtained on the same instrument at 62.5 MHz. Mass spectra were obtained with a Hewlett-Packard 5985 mass spectrophotometer. Column chromatography was performed with Merck 60 brand of silica gel. GLC analyses were obtained with a Hewlett-Packard Model 5840 or a Perkin-Elmer model 920 gas chromatograph, using either a 6 ft., 2-mm i.d. glass column packed with 3% nonpolar silicone on diatomaceous earth, 100-120 mesh or a 10 ft., 2 mm i.d. glass column packed with 2% polyethylene glycol on diatomaceous earth, 100-120 mesh. Where indicated, percentages refer to computer calculated peak areas without correction for response. GLC and mass spectral data were provided courtesy of the Fritzsche, Dodge and Olcott. Inc., Instrumental Laboratory.

Elemental microanalyses were performed by Childers Laboratories, Milford, N.J. or Schwarzkopf Microanalytical Laboratory, Woodside, N.Y. Melting points were determined with a Thomas Model 40 micro hot-stage apparatus and are uncorrected. Optical rotations were obtained in chloroform solution at ambient temperature unless otherwise noted using a Perkin-Elmer 241 polarimeter.

### EXAMPLE 1

### Sclareol Epoxide Derivative

To a mixture of sclareol monoacetate which may be prepared as described by G. Buchi and K. Bieman in Croat. Chem. Acta 1957, 29 163-171 (7.00g, 0.02 mol) vanadium (IV) bis(2,4-pentanedionate) oxide (0.100g, 0.38 mmol) and methylene chloride (60 mL) heated at reflux was added a solution of t-butyl hydroperoxide (3 mL, 0.027 mol, Lucidol 90) in methylene chloride (30 mL) over a 1h period. The mixture was heated at reflux for 2h and then a solution of t-butyl hydroperoxide (1 mL, 0.009 mol) in methylene chloride (15 mL) was added over a 0.5h period. The mixture was heated at reflux for an additional 2h and then stirred at 25°C. A 10% sodium sulfite solution (75 mL) was added and the mixture was stirred for 1.5h at 25°C. The phases were separated and the aqueous phase was extracted with methylene chloride (2 X 25 mL). The combined organic layers were washed with 10% sodium sulfite solution (2 X 30 mL) (negative starch iodide test), saturated sodium bicarbonate solution and dried (Na₂SO₄). Evaporation of solvents provided 7.038 g of a nearly colorless solid. Crystallization from hexane/ethyl acetate gave 6.399g (87% yield) of [1R-(1α-(R*),2β,4aβ,8aα)]-2-acetyloxy-α-oxiranyldecahydro-α,2,5,5, 8a-pentamethyl-1-naphthalenepropanol (IIIb), mp 129.5-131°C; [α]D -35.75 ° (c, 3.43).¹H-NMR (250 MHz, CDCl₃) δ 0.74 (3H, s), 0.79 (3H, s), 0.82 (3H, s), 1.25 (3H, s), 1.41 (3H, s), 1.88 (3H, s). 0.9-1.8 (16H, m), 2.51-2.93 (4H, m); ¹³C-NMR δ 15.68 (q), 18.34 (t), 19.07 (t), 20.01 (t), 20.64 (q), 21.40 (q), 22.79 (q), 25.84 (q), 33.07 (s), 33.28 (q), 38.82 (t), 39.55 (s), 39.66 (t), 41.90 (t), 41.95 (t), 44.03 (t), 55.70 (d), 57.72 (d), 69.41 (s), 88.08 (s), 169.85 (s); IR (CHCl₃) v max 3530, 2940, 1720, 1460, 1385, 1360, 1260 cm⁻¹; MS, m/e 306, 291, 273, 109, 95, 81, 43. Anal. Calcd for C₂₂H₃₈0₄: C, 72.09, H, 10.45. Found: C, 72.17; H, 10.45.

### EXAMPLE 2

### Dihydro Sclareol Derivative

A mixture of sclareol monoacetate (3.50g, 0.01 mol), platinum oxide (0.225g), sodium nitrite (0.01g) and ethanol (40 mL) was shaken under a hydrogen atmosphere (40 psi) for 2.5h. The mixture was filtered through celite and the solids were washed with ethanol. The solvent was evaporated and the residue crystallized from hexane to give 2.69g (76% yield) of [1R-[1α,(S*),2β,4aβ,8aα]]-2-acetyloxy-α-ethyldecahydro-α, 2,5,5,8a-pentamethyl-1-naphthalenepropanol (IIIc), mp 92-94°C, [α]D -27.35° (c, 1.547); (literature references mp 93-94.5°C, see D.B. Bigley, N.A.J. Rogers. J.A. Barltrop in J. Chem. Soc., 1960, 4613-4627 and R.K. Grant, C. Huntrakal, and R.T. Weavers, Aust. J. Chem., 1972, 25 365-74.) ¹H-NMR (250 MHz, CDCl₃)δ0.78 (3H, s), 0.84 (3H, s), 0.87 (3H, s), 0.90 (3H, t, J = 7.5 Hz), 1.16 (3H, s), 1.47 (3H, s), 1.93 (3H, s), 0.8-1.9 (18H, m), 2.60-2.68 (1H, m). IR (CDCl₃) v max 3580, 3450, 2950, 1720, 1455, 1485, 1460, 1260 cm⁻¹. MS, m/e 352, 292, 274, 259, 245, 204, 137, 109, 95, 43.

### EXAMPLE 3

### Sclareol Acetate Derivative

To a mixture of sclareol monoacetate (1.050g, 0.003 mol), iodosobenzene diacetate (0.966g, 0.003 mol), calcium carbonate (1.2g, 0.012 mol) and benzene (60 mL) heated at reflux was added dropwise over a 45 min period a solution of iodine (0.762g, 0.003 mol) in benzene (25 mL). The mixture was heated at reflux for 1.5h, cooled, decanted from solids, washed with 5% sodium thiosulfate solution (2 X 50 mL), saturated sodium bicarbonate solution (2 X 30 mL) and dried (Na₂SO₄). The solvent was evaporated and the residue Kugelrohr distilled (bath 60°C, 0.5 mm) to remove most of the iodobenzene. Chromatography (eluant:hexane:ethyl acetate: 20:1) of the residue gave 0.429g (35% yield) of [1R-(1α,2β,4aβ,8aα)]-decahydro-1-(2-iodoethyl)-2,5,5,8a-tetramethyl-2-naphthalenol acetate, the decalin derivative of formula I wherein X is I and R' is COCH₃. Crystallization from hexane provides a pure sample: mp 94-99°C with decomposition; [α]D - 4.28 (c, 1.33).¹H-NMR (250 MHz, CDCl₃) δ 0.77 (3H, s), 0.81 (3H, s), 0.85 (3H, s), 1.46 (3H, s), 1.93 (3H, s), 0.85-2.1 (13H, m), 2.62-2.73 (1H, m), 3.09-3.35 (2H, m); ¹³C-NMR δ 7.59 (t), 15.78 (q), 18.32 (t), 19.92 (t), 20.48 (q), 21.43 (q), 22.91 (q), 31.84 (t), 33.16 (s), 33.31 (q), 38.95 (t), 39.26 (t), 39.73 (t), 41.83 (t), 55.66 (d), 61.17 (d), 87.26 (s), 169.73 (s); IR (CHCl₃) v max 2940, 1720, 1450, 1385, 1360, 1250 cm⁻¹; MS, m/e 346, 331, 279, 219, 137, 109, 95, 43. Anal. Calcd for C₁₈H₃₁IO₂: C, 53.20; H, 7.69; I, 31.23. Found: C, 53.76; H, 7.99; I, 30.91. The reaction also provides 0.639g (48% yield) of [1R-[1R-(1α,2β,4aβ,8aα)]] -1-(5-iodo-3,4-epoxy-3-methylpentyl)-decahydro-2,5,5,8a-tetra methyl-2-naphthalenol acetate (Xl) as a mixture of isomers. ¹H- NMR (250 MHz, CDCl₃) δ 0.78 (3H, s), 0.81 (3H, s), 0.86 (3H, s), 1.28 and 1.34 (3H, 2s), 1.46 and 1.47 (3H, 2s), 1.930 and 1.932 (3H, 2s), 0.8-2.0 (15H, m), 2.6-2.75 (1H, m), 2.9-3.4 (3H, m); IR (CDCl₃) v max 2930, 1720, 1455, 1385, 1360, 1255 cm⁻¹. MS, m/e 416, 401, 384, 289, 245, 204, 137, 109, 95, 43.

### EXAMPLE 4

### Sclareol Acetate Derivative

To a mixture of lead tetraacetate [5.32g, 0.012 mol, washed with hexane (2 X 50 mL)], calcium carbonate (240g, 0.024 mol) sclareol monoacetate (2.10g, 0.006 mol) and benzene (150 mL) heated at reflux was added a solution of iodine (1.524g, 0.006 mol) in benzene (90 ml) over a 1.5h period. The mixture was heated at reflux for 1h, cooled and filtered. The filtrate was washed with 5% sodium thiosulfate solution (2 X 50 mL), saturated sodium bicarbonate solution, dried (Na₂SO₄) and evaporated. Chromatography (eluant:hexane:ethyl acetate; 20:1) gave 1.209g (50% yield) of iodo-acetate (I; X = I,R'= COCH₃) and 0.906g (32% yield) of iodo-epoxide (XI). The compounds were characterized as described in Example 3.

### EXAMPLE 5

### Decalin Derivative

To a mixture of epoxide (IIIb) (0.732g, 0.002 mol, from Example 1) iodosobenzenediacetate (1.288g, 0.004 mol), calcium carbonate (0.8g, 0.008 mol) and benzene (40 mL) heated at reflux was added dropwise over a 45 min period a solution of iodine (0.508g, 0.002 mol) in benzene (25 mL). The mixture was heated at reflux for 1.5h and cooled. Work-up and chromatography as described in Example 3 provided 0.433g (53% yield) of the decalin derivative of formula I wherein X is I and R' is COCH₃.

### EXAMPLE 6

### Decalin Derivative

To a mixture of lead tetraacetate [2.66g, 0.006 mol, washed with hexane (2 X 30 mL)], calcium carbonate (1.20g, 0.012 mol) benzene (75 mL) and sclareol epoxide derivative of Example I heated at reflux was added a solution of iodine (0.726g, 0.003 mol) in benzene (35 mL) over a 1h period. The mixture was heated at reflux for 1h, cooled and filtered. Work-up and chromatography as described in Example 4 gave 0.665g (55% yield) of the decalin derivative formula I wherein X is I and R' is COCH₃.

### EXAMPLE 7

### Decalin Derivative

To a mixture of lead tetraacetate (3.547g, 0.008 mol), calcium carbonate (1.60g, 0.016 mol), the dihydrosclareol derivative of Example 2 (1.418g, 0.004 mol) and benzene (200 mL) heated at reflux was added a solution of iodine (1.016g, 0.004 mol) in benzene (50 mL) over a 1.5h period. The mixture was heated a reflux for 1h, cooled and filtered. Work-up and chromatography as described in Example 4 gave 0.794 (49% yield) of the decalin derivative of formula I wherein X is I and R is COCH₃ and 0.388g (29% yield) of 13-nor-vinyl-13-ketosclareol acetate which was identical to an authentic sample as reported by D.B. Bigley, N.A.J. Rogers, J.A. Barltrop in J. Chem. Soc., 1960 4613-4627.

### EXAMPLE 8

### Dodecahydro-3a,6,6,9a-tetramethylnaphtho[2,1-b]furan

A mixture of the decalin derivative of formula I wherein X is I and R' is COCH₃ (0.610g, 0.0015 mol from any of Examples 3 through 7), potassium hydroxide (0.593g, 0.009 mol), isopropanol:water (6:1, 50 mL) was heated at reflux for 18h. The mixture was cooled and concentrated under reduced pressure. The residue was added to water and extracted with hexane (4 x 20 mL). The extracts were washed with water (15 mL), brine (15 mL) and dried (Na₂SO₄). The solvents were evaporated and the residue chromatographed (eluant:hexane:ethyl acetate; 20:1). Kugelrohr distillation (bath 120°C, 1 mm) gave 0.286g (81% yield, 99.5% pure according to GLC analysis) of compound II, mp 74.5-76°C, [α]D -29.90° (c, 3.01, benzene); literature reference: mp 75-76°C, [α]D -28.0° (benzene) see M. Stoll and M. Hinder in Helv. Chim. Acta, 1953, 36, 1995-2008; NMR (250 MHz, CDCl₃) δ 0.83 (6H, s), 0.88 (3H, s), 1.08 (3H, s), 0.8-2.0 (14H, m), 3.75-3.96 (2H, m); ¹³C-NMR δ 15.06 (q), 18.47 (t), 20.72 (t), 22.69 (2q), 33.10 (s), 36.27 (s), 39.36 (t), 40.06 (t), 42.54 (t), 57.36 (d), 60.22, (d), 64.94 (t), 79.84 (s); IR (melt) v max 2930, 1480, 1460, 1390, 1375 cm⁻¹; MS, m/e 236, 221, 204, 137, 97.

## Claims (Claims for the following Contracting State(s): AT, BE, CH, DE, FR, GB, GR, IT, LI, LU, NL, SE)

1. A method for the preparation of a decalin derivative of the formula 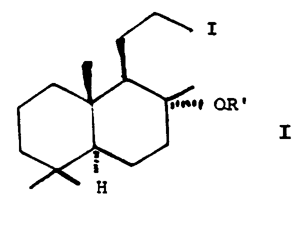 wherein R' is an alkanoyl group of 2 to 5 carbons, which comprises
treating a sclareol alkanoate derivative of the formula 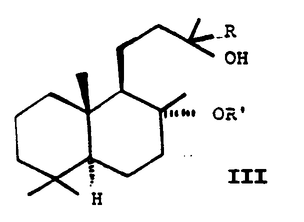 wherein R is vinyl, epoxyethyl or ethyl and R' is an alkanoyl group of 2 to 5 carbons with iodine and an oxidizing agent.

2. A method for the production of dodecahydro-3a,6,6,9a-tetramethyl-naphtho[2,1-b]furan which comprises basically hydrolyzing the decalin derivative of claim 1.

3. A method according to claim 1 wherein the oxidizing agent is mercuric oxide, lead tetraacetate, iodosobenzene diacetate, iodosobenzene, periodinane or iodoxybenzene.

4. A method according to claim 1 wherein the solvent is an aromatic hydrocarbon, aliphatic hydrocarbon or a halocarbon.

5. A method according to claim 1 wherein the temperature used is from about 0°C to about 50°C.

6. A method according to claim 1 wherein the amount of oxidant is from about 50 to about 250 mole percent relative to the number of moles of sclareol acetate derivative present.

7. A method according to claim 6 wherein the amount of oxidant is from about 50 to about 200 mole percent.

8. A method according to claim 6 wherein the amount of oxidant is from about 100 to 200 mole percent.

9. An epoxysclareol acetate of the formula

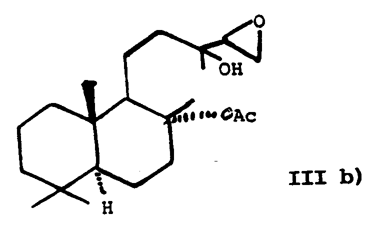

## Claims (Claims for the following Contracting State(s): ES)

1. A method for the preparation of a decalin derivative of the formula 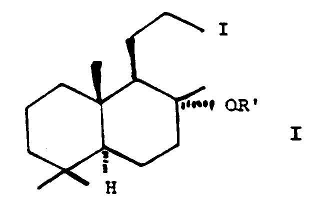 wherein R' is an alkanoyl group of 2 to 5 carbons, which comprises
treating a sclareol alkanoate derivative of the formula 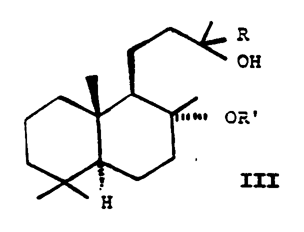 wherein R is vinyl, epoxyethyl or ethyl and R' is an alkanoyl group of 2 to 5 carbons with iodine and an oxidizing agent.

2. A method for the production of dodecahydro-3a,6,6,9a-tetramethyl-naphtho[2,1-b]furan which comprises basically hydrolyzing the decalin derivative of claim 1.

3. A method according to claim 1 wherein the oxidizing agent is mercuric oxide, lead tetraacetate, iodosobenzene diacetate, iodosobenzene, periodinane or iodoxybenzene.

4. A method according to claim 1 wherein the solvent is an aromatic hydrocarbon, aliphatic hydrocarbon or a halocarbon.

5. A method according to claim 1 wherein the temperature used is from about 0°C to about 50°C.

6. A method according to claim 1 wherein the amount of oxidant is from about 50 to about 250 mole percent relative to the number of moles of sclareol acetate derivative present.

7. A method according to claim 6 wherein the amount of oxidant is from about 50 to about 200 mole percent.

8. A method according to claim 6 wherein the amount of oxidant is from about 100 to 200 mole percent.

## Patentansprüche (Patentansprüche für folgende(n) Vertragsstaat(en): AT, BE, CH, DE, FR, GB, GR, IT, LI, LU, NL, SE)

1. Verfahren zur Herstellung eines Decalinderivats der Formel 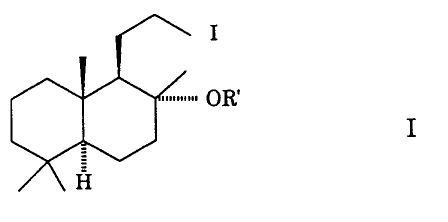 in der R' eine C₂₋₅-Alkanoylgruppe bedeutet,
umfassend Behandeln eines Sclareolalkanoatderivats der Formel 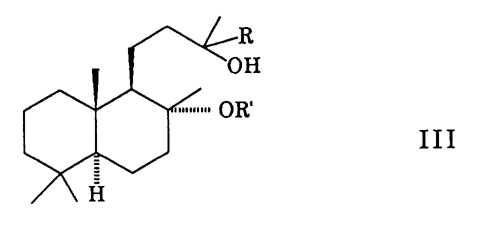 in der R Vinyl, Epoxyethyl oder Ethyl und R' ein Alkanoylrest mit 2 bis 5 Kohlenstoffatomen bedeuten,
mit Jod und einem Oxidans.

2. Verfahren zur Herstellung von Dodecahydro-3a,6,6,9a-tetramethylnaphtho[2,1-b]furan, umfassend basische Hydrolyse des Decalinderivats von Anspruch 1.

3. Verfahren nach Anspruch 1, wobei das oxidierende Agens Quecksilber (II) oxid, Bleitetraacetat, Iodosylbenzoldiacetat, Iodosylbenzol, Periodinan oder Iodoxybenzol ist.

4. Verfahren nach Anspruch 1, wobei das Lösungsmittel ein aromatischer Kohlenwasserstoff, ein aliphatischer Kohlenwasserstoff oder ein halogenierter Kohlenwasserstoff ist.

5. Verfahren nach Anspruch 1, wobei eine Temperatur von etwa 0° C bis etwa 50° C verwendet wird.

6. Verfahren nach Anspruch 1, wobei die Menge des Oxydans etwa 50 bis etwa 250 Mol-%, bezogen auf die Molzahl des vorhandenen Sclareolacetatderivats, beträgt.

7. Verfahren nach Anspruch 6, wobei die Menge des Oxydans etwa 50 bis etwa 200 Mol-% beträgt.

8. Verfahren nach Anspruch 6, wobei die Menge des Oxydans etwa 100 bis etwa 200 Mol-% beträgt.

9. Ein Epoxysclareolacetat der Formel

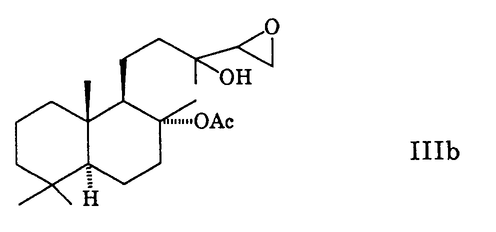

## Patentansprüche (Patentansprüche für folgende(n) Vertragsstaat(en): ES)

1. Verfahren zur Herstellung eines Decalinderivats der Formel 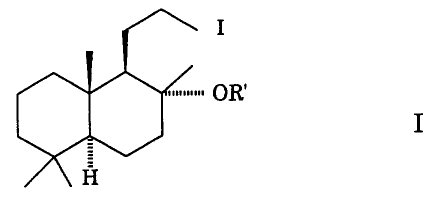 in der R' eine C₂₋₅-Alkanoylgruppe bedeutet,
umfassend Behandeln eines Sclareolalkanoatderivats der Formel 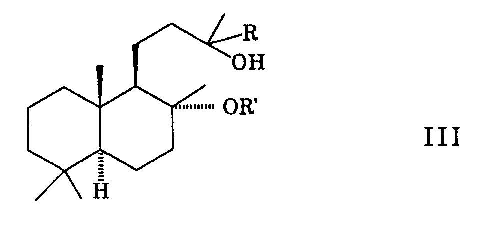 in der R Vinyl, Epoxyethyl oder Ethyl und R' ein Alkanoylrest mit 2 bis 5 Kohlenstoffatomen bedeuten,
mit Jod und einem Oxidans.

2. Verfahren zur Herstellung von Dodecahydro-3a,6,6,9a-tetramethylnaphtho[2,1-b]furan, umfassend basische Hydrolyse des Decalinderivats von Anspruch 1.

3. Verfahren nach Anspruch 1, wobei das oxidierende Agens Quecksilber (II) oxid, Bleitetraacetat, Iodosylbenzoldiacetat, Iodosylbenzol, Periodinan oder Iodoxybenzol ist.

4. Verfahren nach Anspruch 1, wobei das Lösungsmittel ein aromatischer Kohlenwasserstoff, ein aliphatischer Kohlenwasserstoff oder ein halogenierter Kohlenwasserstoff ist.

5. Verfahren nach Anspruch 1, wobei eine Temperatur von etwa 0° C bis etwa 50° C verwendet wird.

6. Verfahren nach Anspruch 1, wobei die Menge des Oxydans etwa 50 bis etwa 250 Mol-%, bezogen auf die Molzahl des vorhandenen Sclareolacetatderivats, beträgt.

7. Verfahren nach Anspruch 6, wobei die Menge des Oxydans etwa 50 bis etwa 200 Mol-% beträgt.

8. Verfahren nach Anspruch 6, wobei die Menge des Oxydans etwa 100 bis etwa 200 Mol-% beträgt.

## Revendications (Revendications pour l'(les) Etat(s) contractant(s) suivant(s): AT, BE, CH, DE, FR, GB, GR, IT, LI, LU, NL, SE)

1. Procédé de préparation d'un dérivé de décaline de formule I 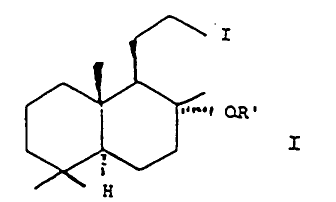 où R' est un groupe alcanoyle de 2 à 5 atomes de carbone, qui comprend
le fait de traiter un dérivé alcanoate de sclaréol de formule 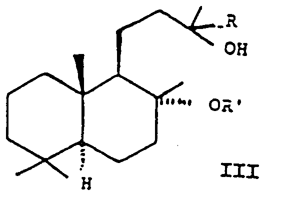 où R est un vinyle, époxyéthyle ou éthyle et R' est un groupe alcanoyle de 2 à 5 atomes de carbone avec de l'iode et un agent oxydant.

2. Procédé de production de dodécahydro3a,6,6,9a-tétraméthyl-naphto[2,1-b]furane qui comprend d'hydrolyser en milieu basique le dérivé de décaline de la revendication 1.

3. Procédé selon la revendication 1, où l'agent oxydant est
l'oxyde mercurique, le tétraacétate de plomb, l'iodosobenzène diacétate, l'iodosobenzène, le périodinane ou l'iodoxybenzène.

4. Procédé selon la revendication 1, où le solvant est un hydrocarbure aromatique, un hydrocarbure aliphatique ou un hydrocarbure halogéné.

5. Procédé selon la revendication 1, où la température utilisée est comprise entre environ 0°C et environ 50°C.

6. Procédé selon la revendication 1, où la quantité d'oxydant est comprise entre environ 50 et environ 250% molaire par rapport au nombre de moles du dérivé acétate de sclaréol présent.

7. Procédé selon la revendication 6, où la quantité d'oxydant est comprise entre environ 50 et environ 200% molaire.

8. Procédé selon la revendication 6, où la quantité d'oxydant est comprise entre environ 100 et 200% molaire.

9. Acétate d'époxysclaréol de formule

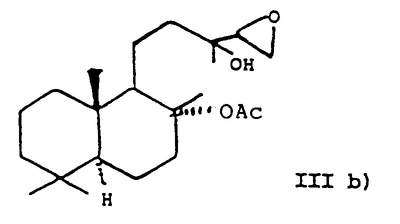

## Revendications (Revendications pour l'(les) Etat(s) contractant(s) suivant(s): ES)

1. Procédé de préparation d'un dérivé de décaline de formule I 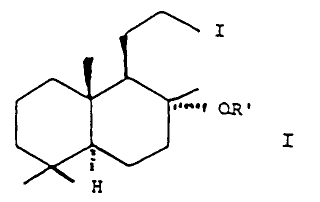 où R' est un groupe alcanoyle de 2 à 5 atomes de carbone, qui comprend
le fait de traiter un dérivé alcanoate de sclaréol de formule 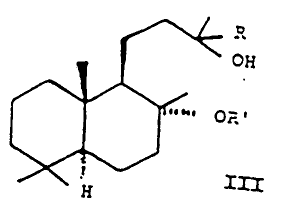 où R est un vinyle, époxyéthyle ou éthyle et R' est un groupe alcanoyle de 2 à 5 atomes de carbone avec de l'iode et un agent oxydant.

2. Procédé de production de dodécahydro3a,6,6,9a-tétraméthyl-naphto[2,1-b]furane qui comprend d'hydrolyser en milieu basique le dérivé de décaline de la revendication 1.

3. Procédé selon la revendication 1, où l'agent oxydant est
l'oxyde mercurique, le tétraacétate de plomb, l'iodosobenzène diacétate, l'iodosobenzène, le périodinane ou l'iodoxybenzène.

4. Procédé selon la revendication 1, où le solvant est un hydrocarbure aromatique, un hydrocarbure aliphatique ou un hydrocarbure halogéné.

5. Procédé selon la revendication 1, où la température utilisée est comprise entre environ 0°C et environ 50°C.

6. Procédé selon la revendication 1, où la quantité d'oxydant est comprise entre environ 50 et environ 250% molaire par rapport au nombre de moles du dérivé acétate de sclaréol présent.

7. Procédé selon la revendication 6, où la quantité d'oxydant est comprise entre environ 50 et environ 200% molaire.

8. Procédé selon la revendication 6, où la quantité d'oxydant est comprise entre environ 100 et 200% molaire.
